# EUROPÄISCHE PATENTANMELDUNG

(11) **EP 2 105 506 A1**
(43) Veröffentlichungstag der Anmeldung: **30.09.2009**
(21) Anmeldenummer: 08005571.8
(22) Anmeldetag: 26.03.2008
(51) Int. Cl.: C12P 7/64

(54) **Verfahren zur Herstellung PUFAs enthaltender Öle unter Verwendung von Mikroorganismen der Ordnung Labyrinthulomycota**

(71) Anmelder: Lonza AG, 4052 Basel (CH)
(72) Erfinder: Die Erfindernennung liegt noch nicht vor

(57) **Zusammenfassung**

Die Erfindung beschreibt ein Verfahren zur fermentativen Gewinnung polyunsaturated fatty acids (PUFAs) enthaltender Öle mit verändertem Fettsäureprofil aus Zellen von Mikroorganismen des Phylums der *Labyrinthulomycota,*
**dadurch gekennzeichnet, dass** dem Fermentationsmedium ein Komplexbildner und/oder ein Flockungsmittel zugegeben wird.

## Beschreibung

Verschiedene mehrfach-ungesättigte Fettsäuren (PUFAs für polyunsaturated fatty acids) und insbesondere omega-3 Fettsäuren (n3-Fettsäuren) sind essentielle Bestandteile der menschlichen Ernährung.

Allerdings ist bekannt, dass in den meisten Industrienationen die Versorgung mit n3-Fettsäuren mangelhaft ist. Dagegen sind der Gesamtfettanteil in der Ernährung sowie die Zufuhr an gesättigten Fettsäuren und N6-Fettsäuren zu hoch. Dies beruht auf einer Veränderung unserer Nahrungszusammensetzung, die vor allem in den letzten ca. 150 Jahren stattgefunden hat und die mit dem Auftreten verschiedener chronischer Zivilisationskrankheiten, wie beispielsweise Herzkreislauferkrankungen - der Haupttodesursache in Industrienationen - korreliert wird (Simopoulos, A.P., 1999, Am. J. Clin. Nutr. 70, 560-569). Eine Vielzahl von Studien hat inzwischen gezeigt, dass durch die gezielte Erhöhung der Zufuhr von n3-Fettsäuren, insbesondere von Eicosapentaensäure (EPA) und von Docosahexaensäure (DHA), das Herzkreislaufrisiko signifikant reduziert werden kann (Dietary supplementation with n-3 polyunsaturated fatty acids and vitamin E after myocardial infarction: results of the GISSI-pevenzione trial., Lancet 354, 447-455; Burr et al., 1989, Effects of changes in fat, fish, and fibre intake on death and myocardial reinfarction: diet and reinfarction trial (DART). Lancet 2, 757-761). Dementsprechend wird von vielen verschieden Organisationen (WHO, FAO, AHA; ISSFAL, British Nutrition Foundation u.v.a.) empfohlen, die Zufuhr von n3-Fettsäuren signifikant zu erhöhen (Kris-Eherton et al., Fish Consumption, Fish Oil, Omega-3 Fatty Acids, and Cardiovascular Disease. Circulation 2002, 2747-2757).

Quellen für die Gewinnung von PUFAs und insbesondere n3-Fettsäuren sind vor allem marine Kaltwasserfische und daraus gewonnene Öle, in den letzten Jahren konnten aber auch marine Mikroorganismen als kommerzielle PUFA-Quellen etabliert werden. Diese haben den Vorteil, dass sie in Fermentern unter kostengünstigen und kontrollierten Bedingungen zur Produktion von PUFAs herangezogen werden können. Bei einer fermentativen Herstellung besteht keine Gefahr der Verunreinigungen (Schwermetalle, Dioxine, PCBs), wie sie für Fische oder daraus gewonnene Fischöle oft beschrieben werden (Olsen SF. Int J Epidemiol. 2001: 1279-80). Außerdem lässt sich durch Auswahl des Organismus ein in seiner Zusammensetzung definiertes Öl herstellen. Die fermentative Herstellung ist zudem keinen saisonalen Schwankungen unterworfen, wie dies ebenfalls für Fisch und Fischprodukte beschrieben ist (Gamez-Meza et al. Lipids 1999:639-42). Darüber hinaus handelt es sich um einen umweltfreundlichen Herstellprozess, da zum einen natürliche Ressourcen geschont werden (Stichwort Überfischung) und zum anderen nachwachsende Rohstoffe in der Fermentation zum Einsatz kommen.

Mikroorganismen, welche sich zur Gewinnung von PUFAs eignen, finden sich beispielsweise bei den Bakterien unter der Gattung *Vibrio* (z. B.: *Vibrio marinus*) oder unter den Dinoflagellaten (*Dinophyta*), dort insbesondere die Gattung *Crypthecodinium*, wie *C. cohnii* oder unter den Stramenopiles, wie die *Pinguiophyceae* wie z.B. *Glossomastix, Phaeomonas, Pinguiochrysis, Pinguiococcus* und *Polydochrysis*. Bevorzugte Mikroorganismen zur fermentativen Herstellung von PUFAs gehören zu den Stramenopiles (oder *Labyrinthulomycota*), insbesondere zur Ordnung *Thraustochytriales*, (*Thraustchytriidea*) und dort wieder insbesondere zu den Gattungen *Japonochytrium, Schizochytrium, Thraustochytrium, Althornia, Labyrinthuloides, Aplanochytrium* und *Ulkenia.*

Es ist bekannt, dass einige der genannten Mikroorganismen zur industriellen Produktion von Fettsäuren verwendet werden können, entsprechende Verfahren wurden beschrieben. So offenbart die internationale Patentanmeldung WO 91/07498 A1 die Herstellung von PUFAs mit Organismen der Gattungen *Schizochytrium* und *Thraustochytrium*. WO 91/11918 A1 offenbart die Herstellung von PUFAs mit *Crypthecodinium cohnii*, WO 96/33263 A1 und die entsprechende europäische Patentanmeldung EP 0 823 475 A1 beschreiben die Herstellung von PUFAs mit Mikroorganismen der Gattung *Schizochytrium*, während die Patentanmeldung WO 98/03671 die Herstellung von PUFAs mit Mikroorganismen der Gattung *Ulkenia* offenbart.

Trotz der hohen Qualität und der anderen oben genannten Vorteile der fermentativen Gewinnung von PUFA-Ölen sind die verhältnismäßig hohen Produktionskosten ein Problem im Vergleich zu einfachen Fischölen. Um die Kosteneffektivität zu verbessern, gibt es eine Reihe von Versuchen, die fermentative Produktivität von PUFA-Ölen zu verbessern. Dies sind vor allem Versuche, die Biomasse-Produktion zu erhöhen, in der Regel durch Zufüttern von Nährstoffen während der Fermentation und/oder verlängerten Fermentationszeiten.

Beides erhöht jedoch auch wieder die Kosten (Rohstoffkosten und/oder Laufzeit der Produktion). Versuche, durch Zusatz von Fermentationshilfsstoffen die Ausbeute zu erhöhen sind ebenfalls beschrieben.. Die US000006410282B1 (WO002001073099A1) beschreibt z.B. die Zugabe von PVP (Polyvinyl Pyrrolidone) zum Medium zur Erhöhung der Ausbeute (DHA bzw. EPA pro Biomasse).

In Anbetracht des Standes der Technik war es daher Aufgabe der vorliegenden Erfindung, ein Verfahren zur gezielten Veränderung des Fettsäureprofils bei gleichzeitig erhöhter Ausbeute bei fermentativer PUFA-Produktion zur Verfügung zu stellen. Ziel war eine qualitative Verbesserung des Öls durch eine erhöhte Konzentration der gewünschten Fettsäuren (PUFA, z.B. DHA, DPA) und eine Reduktion unerwünschter Fettsäuren (z.B. gesättigte Fettsäuren wie Palmitinsäure). Außerdem sollte die Produkt-Konzentration in der Biomasse sowie die Gesamtausbeute (Raum-Zeit-Ausbeute) erhöht werden.

Gelöst werden diese sowie weitere, nicht explizit genannten Aufgaben, die jedoch aus den hierin einleitend diskutierten Zusammenhängen ohne weiteres ableitbar oder erschließbar sind, durch den Gegenstand, der in den Ansprüchen der vorliegenden Erfindung definiert ist.

Ein vorteilhaftes Verfahren für die Kultivierung von Mikroorganismen des Phylums der *Labyrinthulomycota* wird durch das in Anspruch 1 definierte Verfahren zur Verfügung gestellt. Dieses Verfahren umfasst die Kultivierung in einem geeigneten Fermentationsmedium unter Zugabe von bestimmten Eisen- bzw. EDTA-Verbindungen in einer Konzentration von bis zu 0,5 mM und gegebenenfalls die anschließende Isolation der PUFAs aus den Mikroorganismen und/oder dem Kulturmedium.

Die Erfindung umfasst weiterhin ein Verfahren zur Produktion hochreiner PUFAs.

Bevorzugte PUFAs sind erfindungsgemäß DHA, DPA und EPA.

Insbesondere führt das oben genannte Verfahren zu einer Erhöhung der DHA Konzentration in der Biomasse von mehr als 5%, bevorzugt mehr als 10%, und ganz besonders bevorzugt mehr als 15%.

Insbesondere führt das oben genannte Verfahren zu einer Erhöhung der DHA Ausbeute (g/L) von mehr als 5%, bevorzugt mehr als 10%, und ganz besonders bevorzugt mehr als 15%.

Insbesondere führt das oben genannte Verfahren zu einer Erhöhung der DHA Konzentration im Öl von mehr als 2%, bevorzugt mehr als 3%, und ganz besonders bevorzugt mehr als 4%.

Insbesondere führt das oben genannte Verfahren zu einer Reduktion der Palmitinsäure (PA) Konzentration im Öl von mehr als 2%, bevorzugt mehr als 3%, und ganz besonders bevorzugt mehr als 4%.

Dabei wird das Verhältnis von DHA zu PA um mehr als 4%, bevorzugt mehr als 6%, und ganz besonders bevorzugt mehr als 8% erhöht.

Insgesamt ermöglicht das oben genannte Verfahren in einem Schritt gleichzeitig die Quantität (Produktivität) und die Ölqualität (erhöhte DHA Konzentration, verringerte Konzentration PA (gesättigte Fettsäure) zu verbessern.

Die Möglichkeit, durch Zugabe von geringen Mengen bestimmter Eisen- bzw. EDTA-Verbindungen das Fettsäureprofil sowie gleichzeitig die Konzentration des Zielproduktes in der Biomasse und die Gesamtausbeute in der Fermentation so deutlich positiv zu beeinflussen, war völlig überraschend.

Durch an die Kultivierung anschließende Isolation der PUFAs aus den Mikroorganismen (Biomasse) und/oder der wässrigen Kultur können die PUFAs in hoher Ausbeute und Reinheit gewonnen werden.

Weiterhin umfasst die vorliegende Erfindung auch ein Verfahren zur Herstellung von Biomasse, wobei die Biomasse durch das erfindungsgemäße Kultivierungsverfahren zur Verfügung gestellt wird.

Diese Biomasse kann auf alle erdenklichen Arten Verwendung finden. Insbesondere kann diese Biomasse, z.B. in getrocknetem Zustand (Biotrockenmasse), direkt als Nahrungsmittel oder als Futtermittel eingesetzt werden.

Weiterhin umfasst die Erfindung auch ein Öl, welches dadurch gewonnen wird, dass das erfindungsgemäße Kultivierungsverfahren durchgeführt wird, und das Öl aus den Mikroorganismen und/oder der wässrigen Kultur isoliert wird.

Insbesondere handelt es sich dabei um ein Öl, welches neben vielen weiteren bevorzugten Verwendungszwecken mit Vorteil für die menschliche Ernährung verwendet werden kann.

Unter Öl wird erfindungsgemäß bevorzugt ein Anteil von mindestens 70 % Neutrallipiden und mindestens 2% Phospholipiden verstanden, was dem normalen, dem Fachmann bekannten Fettsäurespektrum der *Thraustochytriales* entspricht. Die Neutrallipide bestehen dabei bevorzugt aus mindestens 80% Triglyceriden und anderen Verbindungen wie z.B. Diacylglyzeride, Sterole usw. Weiterhin besteht der Gewichtsanteil der Triglyzeride bevorzugt aus etwa 95% Fettsäuren und 5% Glyzerin.

PUFAs sind erfindungsgemäß mehrfach ungesättigte langkettige Fettsäuren mit einer Kettenlänge >C12 mit mindestens zwei Doppelbindungen. Nach dem erfindungsgemäßen Verfahren herstellbare PUFAs sind insbesondere n3-Fettsäuren und n6-Fettsäuren.

Unter n3-Fettsäuren (omega-3 Fettsäure, ω3 Fettsäuren) im erfindungsgemäßen Sinn werden mehrfach ungesättigte langkettige Fettsäuren mit einer Kettenlänge >C12 mit mindestens zwei oder mehr Doppelbindungen verstanden, wobei die erste der Doppelbindungen zwischen den Kohlenstoffatomen C3 und C4 ausgehend vom Alkylende konstituiert ist. Dementsprechend liegt bei n6-Fettsäuren die erste Doppelbindung zwischen den Kohlenstoffatomen C6 und C7 ausgehend vom Alkylende.

Für die Produktion der PUFAs nach dem erfindungsgemäßen Verfahren kommen Mikroorganismen aus dem Phylum der Labyrinthulomycota in Frage. Mikroorganismen der Ordnung *Thraustochytriales* (*Thraustochytriidea*) sind bevorzugt (Lewis, T.E., Nichols, P.D., McMeekin, T.A., The Biotechnological Potential of Thraustochytrids, Marine Biotechnology, 1999, S. 580-587 und Porter, D. Phylum Labyrinthulomycota in Handbook of protoctista; Editors: Margulis, L, Corliss, J.O., . Melkonian, M. and Chapman, D.J., Jones and Bartlett Publishers, ISBN 0-86720-052-9 1990, S. 388-398). Besonders bevorzugt sind Mikroorganismen der Gattungen *Japonochytrium, Schizochytrium, Thraustochytrium Althornia, Labyrinthuloides, Aplanochytrium* und *Ulkenia.* Ganz besonders bevorzugt sind hiervon *Schizochytrium, Thraustochytrium* und *Ulkenia.* Insbesondere bevorzugt sind: *Japonochytrium* sp. ATCC 28207, *Thraustochytrium aureum* (insbesondere ATCC 28211 bzw. ATTC 34304), *Thraustochytrium roseum* ATCC 28210 *Thraustochytrium* sp. ATCC 20890, ATTC 20891, ATTC 20892 und ATTC 26185, *Schizochytrium aggregatum* ATTC 28209, *Schizochytrium* sp. ATCC 20888 und ATTC 20889, *Schizochytrium* SR21, sowie *Ulkenia* spec. SAM 2179 und SAM 2180.

Für das erfindungsgemäße Verfahren geeignete Mikroorganismen sind sowohl Wildtyp-Formen als auch Mutanten und daraus abgeleitete Stämme sowie rekombinante Stämme der entsprechenden Organismen. In besonderem Maße umfasst die vorliegende Erfindung Mutanten oder rekombinante Stämme zur Produktionssteigerung von PUFA.

Die Mikroorganismen im Sinne der vorliegenden Erfindung werden durch Animpfen eines flüssigen oder eines festen Mediums mit einer Vorkultur dieser Organismen kultiviert.

Für Mikroorganismen des Phylums der *Labyrinthulomycota* geeignete Kulturtechniken sind dem Fachmann gut bekannt. Typischerweise, aber nicht ausschließlich, wird die Kultur mittels wässriger Fermentation in einem entsprechenden Behältnis durchgeführt. Beispiele für typische Behältnisse für eine derartige Fermentation umfassen Schüttelkolben oder Bioreaktoren, wie beispielsweise STRs (stirred tank reactors) oder Biasensäuien. Die Kultur wird typischerweise bei Temperaturen zwischen 10°C und 40°C durchgeführt, bevorzugterweise zwischen 20°C und 35 °C, besonders bevorzugt zwischen 25°C und 30°C, ganz besonders bevorzugt zwischen 27°C und 29°C und insbesondere bei 28 ±0.5°C.

Neben den für das Wachstum notwendigen eigentlichen Nährstoffen werden das Wachstum und die Entwicklung von allen Lebewesen durch die Zufuhr von Mineralstoffen, Vitaminen und Spurenelementen beeinflusst. Es wird auch zwischen Makroelementen (die in großer Menge gebraucht werden) und Mikroelementen (die nur in geringer Menge gebraucht werden) unterschieden. Ein Mangel an Makroelementen macht sich sehr schnell bemerkbar, während sich ein Mangel an Mikroelementen oft erst nach längerer Zeit bemerkbar macht. Bei längeren Defiziten ergeben sich Mangelerscheinungen.

Es gilt das Gesetz vom Minimum (nach Liebig): Der im Minimum befindliche Faktor bestimmt das Wachstum.

Bei Versuchen mit verschiedenen Salzen konnte beobachtet werden, dass insbesondere durch den Einsatz von Na-EDTA, Fe-Na-EDTA, K-EDTA und Fe-III-Sulfat das Wachstum und die Fettsäurezusammensetzung von PUFAproduzierenden Mikroorganismen gezielt modifiziert werden kann.

Bei Konzentrationen von bis zu 0.5 mM der entsprechenden Eisen- bzw. EDTA Verbindungen konnten positive Veränderungen erzielt werden. Als positiv erwiesen sich dabei Fe-Na-EDTA, Na-EDTA, K-EDTA und Fe-III-SO₄. Als erfindungsgemäße Eisen- bzw. EDTA-Verbindungen seien deshalb Fe-Na-EDTA, Na-EDTA, K-EDTA und FE-III-SO₄ genannt.

In einer bevorzugten Ausführungsform der vorliegenden Erfindung wird dem Medium bis zu 0.5 mM, bevorzugt von 0.05-0.5 mM und besonders bevorzugt von 0.1 bis 0.4 mM einer erfindungsgemässen Eisen- bzw. EDTA-Verbindung zugesetzt. Ganz besonders bevorzugt ist ein Gehalt von 0.2 ±0.1 mM einer erfindungsgemässen Eisen- bzw. EDTA-Verbindung.

Das Medium umfasst ferner bevorzugterweise eine oder mehrere Kohlenstoffquellen, sowie eine oder mehrere Stickstoffquellen. Dem Fachmann sind für die Kultivierung von Mikroorganismen des Phylums der *Labyrinthulomycota* als Kohlenstoff- und Stickstoffquellen verwendbare Substanzen gut bekannt.

Erfindungsgemäss verwendbare Kohlenstoffquellen sind beispielsweise Kohlenhydrate wie Glukose, Fruktose, Xylose, Saccharose, Maltose, lösliche Stärke, Fucose, Glucosamin, Dextran, Glutaminsäure, Melasse, Glyzerin oder Mannitol oder auch Fette und Öle oder pflanzliche Hydrolysate. Ebenso fallen hierunter . Maisquellwasser und Malzextrakt.

Erfindungsgemäß einsetzbare natürliche Stickstoffquellen sind beispielsweise Pepton, Hefeextrakt, Fleischextrakt, Proteinhydrolysate, oder Sojabohnen, einsetzbare organische Stickstoffquellen sind beispielsweise Glutamat und Harnstoff, aber auch anorganische Stickstoffquellen wie zum Beispiel Ammoniumacetat, Ammoniumhydrogencarbonat, Ammoniumsulfat oder Ammoniumnitrat können als Stickstoffquelle verwendet werden.

Das Medium kann alle weiteren dem Fachmann für die Kultivierung von Mikroorganismen des Phylums der *Labyrinthulomycota* förderlichen Bestandteile enthalten, insbesondere anorganische Salze von beispielsweise Ca, Mg, Na, K, Fe, Ni, Co, Cu, Mn, Mo oder Zn. Beispielhaft seien Phosphate wie Kaliumdihydrogenphosphat oder Chloride wie Magnesiumchlorid und Sulfate wie Ammoniumsulfat,' Magnesiumsulfat, Eisensulfat oder Natriumsulfat genannt. Weitere verwendbare anorganische Salze sind beispielsweise Halogenide, wie Kaliumbromid oder Kaliumjodid und ebenso weitere Carbonate wie zum Beispiel Natriumhydrogencarbonat.

Ggf. kann das Medium zusätzliche Makro- oder Mikronährstoffe, wie Aminosäuren, Purine, Pyrimidine, Corn Steep Liquor (Maisquellwasser), Proteinhydrolysate, Vitamine (wasserlöslich und/oder wasserunlöslich) und andere dem Fachmann gut bekannte Medienbestandteile umfassen. Antischaummittel können, falls notwendig, zugegeben werden. Das Medium kann komplexe Bestandteile enthalten oder chemisch definiert sein.

Die Menge der einzelnen Komponenten kann variieren, solange kein negativer Effekt auf das Wachstum oder die Produktivität der Mikroorganismen vorliegt. Der Fachmann kann die Zusammensetzung entsprechend den Bedürfnissen des Mikroorganismus im Einzelfall leicht ermitteln. Im Allgemeinen wird die Kohlenstoffquelle in einer Konzentration bis 300 g/l und die Stickstoffquelle in einer Konzentration von 1 bis 30 g/l zugegeben. Vorzugsweise wird der Stickstoffgehalt in Abhängigkeit vom Kohlenstoffgehalt des Mediums gestellt.

Ein besonders bevorzugtes Medium umfasst Glukose, Hefeextrakt, Maisquellwasser (Corn Steep Liqour [CSL]), Magnesiumchlorid, Calciumcarbonat, Calciumchlorid, Natriumsulfat und Kaliumphosphat sowie die genannten Eisen- oder EDTA-Verbindungen.

Der pH-Wert des Mediums wird vor Beginn der Fermentation auf einen Bereich von 3 bis 10, bevorzugt 4 bis 8, besonders bevorzugt 5 bis 7 und ganz besonders bevorzugt etwa 6 durch Zugabe einer entsprechenden Säure oder Lauge eingestellt.

Anschließend wird das Medium sterilisiert. Techniken zur Sterilisation von Medien sind dem Fachmann gut bekannt, beispielhaft seien Autoklavieren und Sterilfiltration genannt.

Die Kultivierung kann in Batch-, Fed-Batch- oder in kontinuierlicher Weise erfolgen, wie es dem Fachmann allgemein bekannt ist.

Eine Batch- oder Fed-Batch-Kultivierung erfolgt üblicherweise über 1 bis 12 Tage, bevorzugt 2-10 Tage, besonders bevorzugt über 3-9 Tage.

Die Medienbestandteile können dem Medium einzeln oder vermischt zugegeben werden, auch eine vorgefertigte Mischung ist denkbar. Die Bestandteile, insbesondere die Kohlenstoff- und Stickstoffquelle(n) oder bestimmte Zusätze können vor oder während der Kultivierung zugegeben werden. Die Zugabe kann einmal, mehrmals wiederholt oder auch kontinuierlich erfolgen.

Die produzierten PUFA liegen im Allgemeinen in Form von Neutralfetten zum Beispiel als Triacylglyzeride oder polare Lipide wie zum Beispiel Phosphatidylcholin, Phosphatidylethanolamin oder Phosphatidylinositol vor.

PUFA-haltige Öle können nach der Fermentation durch Pressen, durch Einsatz von Lösemitteln wie Hexan, Aceton, Ethanol etc oder durch Zellaufbruch und Separation gewonnen werden. Die so gewonnen Öle können weiteren typischen

Verarbeitungsschritten wie Bleichung, Neutralisation, Entschleimung, Deodorierung etc (Raffination) unterzogen werden. Die PUFA-reiche Biomasse kann aber auch direkt verwendet werden, z.B. zur Tierernährung.

Für die Zwecke der vorliegenden Erfindung werden unter den Begriffen PUFA, n3-Fettsäure oder n3-Wirkstoffe alle möglichen chemischen Formen verstanden, in denen die entsprechenden Fettsäuren vorliegen können, d. h. sowohl als freie Fettsäuren, Ester, Triglyceride, Phospholipide als auch als andere Derivate. All diese Substanzen werden im Folgenden zusammengefasst und die Begriffe werden synonym verwendet. Im weiteren können die PUFAs durch chemische oder biokatalytische Umesterung beispielsweise mit Hilfe geeigneter Enzyme (Lipasen) umgesetzt und angereichert werden, vor oder nach der Isolierung aus der Kultur.

Die Isolierung von PUFAs aus den fermentierten Mikroorganismen bzw. dem Medium und die Analyse des Fettsäurespektrums erfolgt nach für den Fachmann bekannten und üblichen Verfahren (AOCS Methode Ce 1-62 (American Oil Chemists' Society); Wanasundara, U.N., Wanasundara, J., Shahidi, F., Omega-3 fatty acid concentrates: a review of production technologies, Seafoods - Quality, Technology and Nutraceutical Applications, 2002, S. 157-174; Kang and Wang, A simplified method for analysis of polyunsaturated fatty acids, BMC Biochemistry 2005, 6:5 doi:10.1186/1471-2091-6-5).

Nachfolgend wird das erfindungsgemäßen Verfahren zur gezielten Modifikation des Fettsäureprofils bzw. Ausbeutesteigerung anhand einiger Beispiele beschrieben. Das Fermentationsmedium sowie die Erfindung sind jedoch nicht auf diese Beispiele beschränkt.

**Beispiel 1: Fermentation von Ulkenia spec. Stamm SAM 2179 zur Produktion von PUFA unter Zugabe unterschiedlicher Mengen Fe-Na-EDTA.**

| *Ulkenia* spec. Stamm SAM 2179 wurde in 300ml Erlenmeyerkolben mit einer Schikane in 50mL Medium kultiviert. | |
|---|---|
| | |
| Medienzusammensetzung: | |
| Glucose | 150 g/L |
| Maisquellwasser (CSL) | 3.75 g/L |
| KH₂PO₄ | 3 g/L |
| NaCl | 0.8 g/L |
| MgSO₄ x 7H₂O | 1.5 g/L |
| CaCl₂ x 2H₂O 0.3 | g/L |
| (NH₄)₂SO₄ | 5 g/L |
| CaCO₃ | 7.5 g/L |

Dem Medium wurden unterschiedliche Mengen (0 - 2 ml pro 50 ml) einer Fe-Na-EDTA-Lösung (3 g/L; 8.2 mM) zugesetzt.

Der pH-Wert wurde mit NaOH auf 6.0. eingestellt und das Medium anschließend autoklaviert.

| Kulturbedingungen : | |
|---|---|
| | |
| Temperatur (°C): | 28 |
| Schüttelrate (rpm): | 150 |

Die Zellernte erfolgte nach 96h Kultivierung durch Zentrifugation. Anschließend wurden die Zellen gefriergetrocknet und die Biotrockenmasse bestimmt. Aufschluss der Zellen und Bestimmung der Fettsäuren erfolgte durch Hitzebehandlung für 2 stunden in 10%iger methanolischer Salzsäure bei 60°C (unter Rühren). Die Ester wurden dann im Gaschromatographen zur Bestimmung der Fettsäurezusammensetzung analysiert (AOCS Methode Ce 1-62 (American Oil Chemists' Society; www.aocs.org).

**Tabelle 1 (JV39): Produktivität in Abhängigkeit von der Fe-Na-EDTA-Konzentration (Mittelwerte aus jeweils 3 Kolben).**

| **Fe-EDTA [mM]** | **BTM [g/L]** | **DHA-Area [%]** | **DPA- Area [%]** | **PA-Area [%]** | **DHA/BTM [%]** | **DHA/PA [mg/L]** | **DHA/PA** | **DHA/DPA** |
|---|---|---|---|---|---|---|---|---|
| 0.000 | 56.21 | 43.8 | 10.8 | 34.3 | 18.59 | 10453.01 | 1.28 | 4.08 |
| 0.066 | 54.25 | 45.2 | 11.2 | 32.3 | 20.00 | 10850.20 | 1.40 | 4.03 |
| 0.131 | 54.04 | 45.4 | 11.1 | 32.2 | 18.38 | 9935.06 | 1.41 | 4.10 |
| 0.197 | 51.79 | 45.6 | 10.8 | 32.3 | 18.84 | 9763.69 | 1.41 | 4.23 |
| 0.262 | 52.51 | 45.7 | 11.0 | 32.4 | 22.48 | 11806.16 | 1.41 | 4.16 |
| 0.328 | 53.16 | 45.0 | 11.1 | 32.5 | 17.89 | 9516.26 | 1.39 | 4.05 |

| | | | | | | | | |
|---|---|---|---|---|---|---|---|---|
| BTM: Biotrockenmasse; DHA-Area (%) Anteil DHA im Fettsäurespektrum; DPA-Area (%) Anteil DPA im Fettsäurespektrum; PA-Area (%) Anteil Palmitinsäure im Fettsäurespektrum; DHA/BTM: Gew.-% DHA (Docosahexaensäure) pro | | | | | | | | |

Gewichtseinheit BTM; DHA [mg/L] = Ausbeute DHA in mg pro Liter Fermentationsansatz; DHA/PA und DHA/DPA = Verhältnis der Fettsäuren

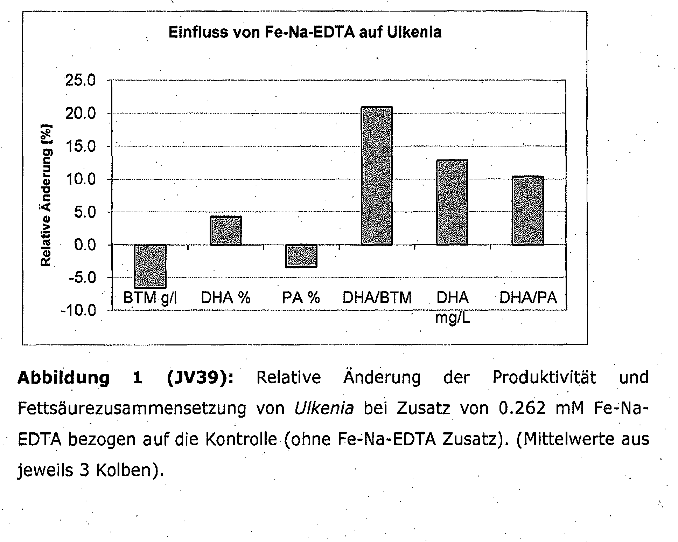

Der Zusatz von Fe-Na-EDTA im Fermentationsmedium führt bei *Ulkenia* spec. SAM 2179 zu einer Veränderung des Fettsäureprofils sowie einem erhöhten Gehalt an DHA pro Biomasse und einer erhöhten Gesamtausbeute. Bei 0.262 mM Fe-Na-EDTA beträgt die Veränderung des Fettsäurespektrums +4.3% für DHA und -3.4% für PA. Das Verhältnis DHA/PA steigt um 10.4%. Gleichzeitig steigt der Gehalt an DHA in der Biomasse um 20.9%. Dies führt insgesamt trotz einer Abnahme der Biomasse zu einer 12.9% höheren DHA Ausbeute (g/L). Es kann also sowohl eine qualitative Verbesserung des Öls (Fettsäurespektrum) als auch eine quantitative Verbesserung erzielt werden (Tab. 1 und Abb. 1).

**Beispiel 2**: Fermentation von *Ulkenia* spec. Stamm SAM 2179 zur Produktion von PUFA unter Zugabe unterschiedlicher Mengen Na-EDTA.

Die Kultivierung erfolgte wie unter Bsp. 1 beschrieben unter Zusatz von Na-EDTA.

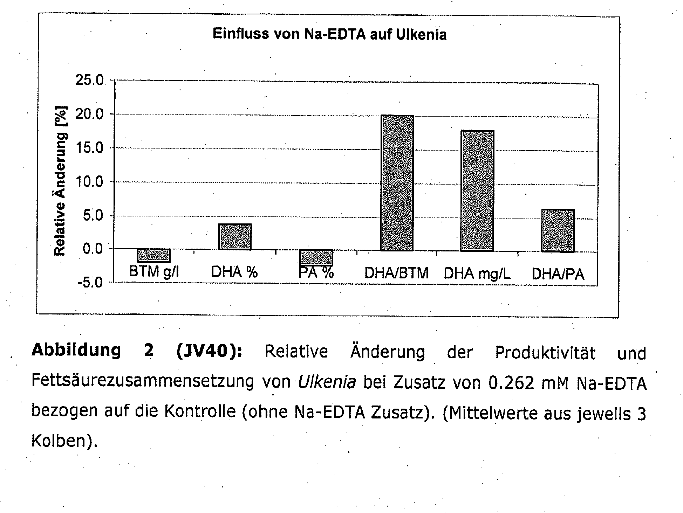

Der Zusatz von Na-EDTA im Fermentationsmedium führt zu ähnlichen Veränderungen im Fettsäureprofil, DHA-Gehalt pro Biotrockenmasse und Gesamtausbeute wie Fe-Na-EDTA. Bei 0.262 mM Na-EDTA beträgt die Veränderung des Fettsäurespektrums +3.8% für DHA und -2.3% für PA. Das Verhältnis DHA/PA steigt um 6,3%. Gleichzeitig steigt der Gehalt an DHA in der Biomasse um 20.1%. Dies führt insgesamt trotz einer Abnahme der Biomasse zu einer 17.9% höheren DHA Ausbeute (g/L). Es kann also sowohl eine qualitative Verbesserung des Öls (Fettsäurespektrum) als auch eine quantitative Verbesserung erzielt werden (Tab. 2 und Abb. 2).

**Tabelle 2 (JV40): Produktivität in Abhängigkeit von der Na-EDTA-Konzentration (Mittelwerte aus jeweils 3 Kolben).**

| **Na-EDTA [mM]** | **BTM [g/L]** | **DHA- Area [%]** | **DPA- [%]** | **PA-Area [%]** | **DHAIBTM [%]** | **DHA [mg/L]** | **DHAI PA** | **DHA/DPA** |
|---|---|---|---|---|---|---|---|---|
| 0.000 | 58.1 | 43.6 | 10.6 | 34.5 | 19.1 | 11082.5 | 1.26 | 4.10 |
| 0.262 | 57.0 | 45.2 | 10.7 | 33.7 | 22.9 | 13060.4 | 1.34 | 4.27 |

| | | | | | | | | |
|---|---|---|---|---|---|---|---|---|
| BTM: Biotrockenmasse; DHA-Area (%) Anteil DHA im Fettsäurespektrum; DPA-Area (%) Anteil DPA im Fettsäurespektrum; PA-Area (%) Anteil Palmitinsäure im Fettsäurespektrum; DHA/BTM: Gew.-% DHA (Docosahexaensäure) pro Gewichtseinheit BTM; DHA [mg/L] = Ausbeute DHA in mg pro Liter Fermentationsansatz; DHA/PA und DHA/DPA = Verhältnis der Fettsäuren | | | | | | | | |

**Beispiel 3:** Fermentation von *Ulkenia* spec. Stamm SAM 2179 zur Produktion von PUFA unter Zugabe unterschiedlicher Mengen von Fe-III-SO₄.

Die Kultivierung erfolgte wie unter Bsp. 1 und 2 beschrieben unter Zusatz von Fe-III-SO₄.

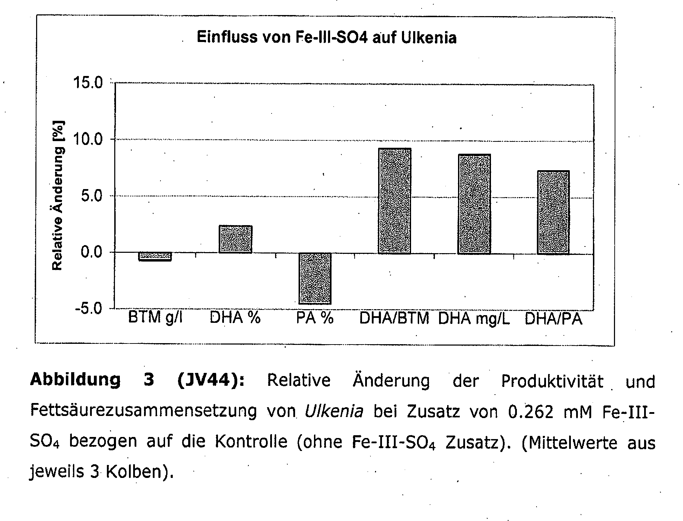

Der Zusatz von Fe-III-SO₄ im Fermentationsmedium führt zu ähnlichen Veränderungen im Fettsäureprofil, DHA-Gehalt pro Biotrockenmasse und Gesamtausbeute wie Fe-Na-EDTA und Na-EDTA. Bei 0.262 mM Fe-III-SO₄ beträgt die Veränderung des Fettsäurespektrums +2.4% für DHA und -4.5% für PA. Das Verhältnis DHA/PA steigt um 7.4%. Gleichzeitig steigt der Gehalt an DHA in der Biomasse um 9.3%. Dies führt insgesamt trotz einer Abnahme der Biomasse zu einer 8.8% höheren DHA Ausbeute (g/L). Obwohl die Effekte mit Fe-III-SO₄ niedriger sind, kann auch hiermit sowohl eine qualitative Verbesserung des Öls (Fettsäurespektrum) als auch eine quantitative Verbesserung erzielt werden (Tab. 3 und Abb. 3).

**Tabelle 3 (JV44): Produktivität in Abhängigkeit von der Fe-III-Sulfat-Konzentration (Mittelwerte aus jeweils 3 Kolben).**

| **Fe-III-SO4 [mM]** | **BTM [g/L]** | **DHA- [%]** | **DPA- Area [%]** | **PA-Area [%]** | **DHA/BTM [%]** | **DHA [mg/L]** | **DHA/PA** | **DHA/DP**A |
|---|---|---|---|---|---|---|---|---|
| 0.000 | 58.1 | 43.7 | 10.7 | 35.7 | 19.7 | 11415.5 | 1.22 | 4.08 |
| 0.262 | 57.7 | 44.7 | 11.0 | 34.1 | 21.5 | 12416.0 | 1.31 | 4.05 |

| | | | | | | | | |
|---|---|---|---|---|---|---|---|---|
| BTM: Biotrockenmasse; DHA-Area (%) Anteil DHA im Fettsäurespektrum; DPA-Area (%) Anteil DPA im Fettsäurespektrum; PA-Area (%) Anteil Palmitinsäure im Fettsäurespektrum; DHA/BTM: Gew.-% DHA (Docosahexaensäure) pro Gewichtseinheit BTM; DHA [mg/L] = Ausbeute DHA in mg pro Liter Fermentationsansatz; DHA/PA und DHA/DPA = Verhältnis der Fettsäuren | | | | | | | | |

**Beispiel 4:** Fermentation von *Ulkenia* spec. Stamm SAM 2179 zur Produktion von PUFA unter Zugabe von K-EDTA

Die Kultivierung erfolgte wie unter Bsp. 1 bis 3 beschrieben unter Zusatz von K-EDTA.

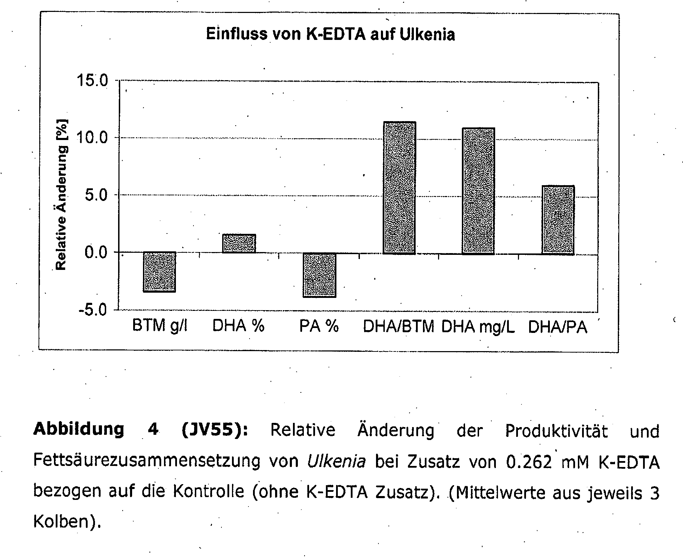

Der Zusatz von K-EDTA im Fermentationsmedium führt zu ähnlichen Veränderungen im Fettsäureprofil, DHA-Gehalt pro Biotrockenmasse und Gesamtausbeute wie Fe-Na-EDTA, Na-EDTA und Fe-III-Sulfat. Bei 0.262 mM K-EDTA beträgt die Veränderung des Fettsäurespektrums +1.6% für DHA und - 3.95% für PA. Das Verhältnis DHA/PA steigt um 5.8%. Gleichzeitig steigt der Gehalt an DHA in der Biomasse um 11.5%. Dies führt insgesamt trotz einer Abnahme der Biomasse zu einer 11% höheren DHA Ausbeute (g/L). Wie bei den vorherigen Experimenten zeigt sich auch hier sowohl eine qualitative Verbesserung des Öls (Fettsäurespektrum) als auch eine quantitative Verbesserung (Tab. 4 und Abb. 4).

**Tabelle 4 (JV55): Produktivität in Abhängigkeit von der K-EDTA-Konzentration (Mittelwerte aus jeweils 3 Kolben).**

| **K-EDTA [mM]** | **BTM [g/L]** | **DHA-Area [%]** | **DPA-Area [%]** | **PA-Area [%]** | **DHA/BTM [%]** | **DHA [mg/L]** | **DHA/PA** | **DHA/DPA** |
|---|---|---|---|---|---|---|---|---|
| 0.000 | 54.6 | 43.8 | 9.7 | 36.1 | 18.5 | 10081.9 | 1.21 | 4.51 |
| 0.262 | 54.3 | 44.5 | 10.5 | 34.7 | 20.6 | 11192.1 | 1.28 | 4.22 |

| | | | | | | | | |
|---|---|---|---|---|---|---|---|---|
| BTM: Biotrockenmasse; DHA-Area (%) Anteil DHA im Fettsäurespektrum; DPA-Area (%) Anteil DPA im Fettsäurespektrum; PA-Area (%) Anteil Palmitinsäure im Fettsäurespektrum; DHA/BTM: Gew.-% DHA (Docosahexaensäure) pro Gewichtseinheit BTM; DHA [mg/L] = Ausbeute DHA in mg pro Liter Fermentationsansatz; DHA/PA und DHA/DPA = Verhältnis der Fettsäuren | | | | | | | | |

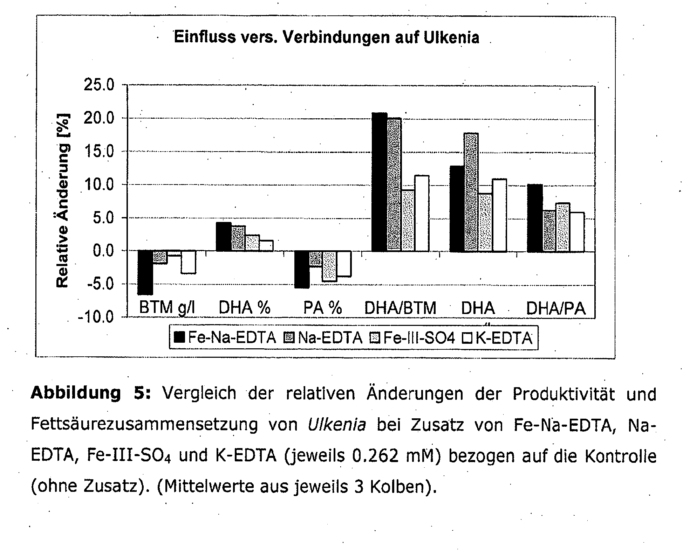

Abbildung 5 zeigt einen Gesamtvergleich der relativen Änderungen der Produktivität und Fettsäurezusammensetzung von Ulkenia bei Zusatz von Fe-Na-EDTA, Na-EDTA, Fe-III-SO₄ und K-EDTA (jeweils 0,262 mM) bezogen auf die Kontrolle.

**Beispiel 5:** Fermentation von *Schizochytrium* SR21 zur Produktion von PUFA unter Zugabe unterschiedlicher Mengen Fe-Na-EDTA, Na-EDTA, oder Fe-III-SO4

Das in der Erfindung beschriebene Verfahren führt auch bei anderen Organismen der *Labyrinthulomycota* zu einer Optimierung der Produktion von PUFA. So kann beispielsweise der Mikroorganismus *Schizochytrium* spec. Stamm SR21 in dem der Erfindung zugrunde liegenden Medium fermentiert werden.

Die Kultivierung erfolgte wie unter Bsp. 1-4 beschrieben unter Zusatz der entsprechenden Fe-bzw. EDTA-Verbindungen.

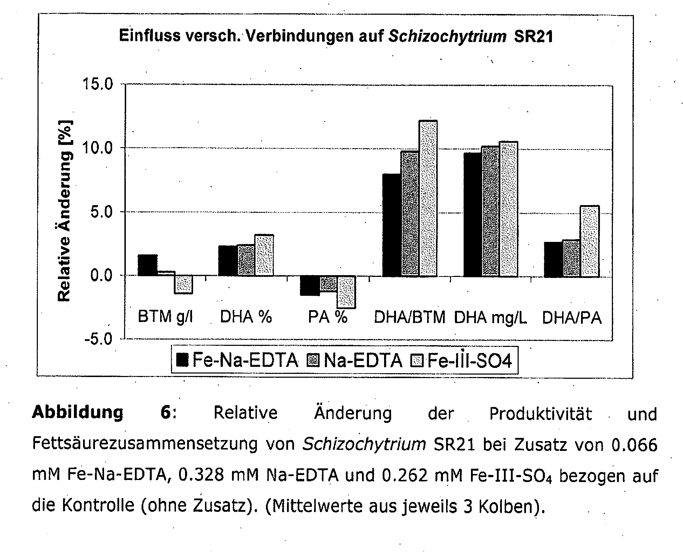

Der Zusatz der genannten Eisen- bzw. EDTA-Verbindungen im Fermentationsmedium führt bei *Schizochytrium* SR21 zu ähnlichen Veränderungen im Fettsäureprofil, DHA-Gehalt pro Biotrockenmasse und Gesamtausbeute wie bei *Ulkenia.* Die Effekte sind allerdings etwas niedriger. Die Veränderung des Fettsäurespektrums beträgt 2.3-3.4% für DHA und -1.2 bis -2.5% für PA. Das Verhältnis DHA/PA steigt um 2.7-5.6%. Gleichzeitig steigt der Gehalt an DHA in der Biomasse um 8-12.2% und die DHA Ausbeute (g/L) um 9.7-10.6%. Es kann also auch bei *Schizochytrium* SR21 sowohl eine qualitative Verbesserung des Öls (Fettsäurespektrum) als auch eine quantitative Verbesserung erzielt werden (Abb. 6). Dieses Beispiel zeigt, dass das der Erfindung zugrunde liegende Verfahren der Zugabe von Eisen- bzw. EDTA-Verbindungen auch bei weiteren Mitgliedern der *Labyrinthulomycota* zu einer Verbesserung der Fermentationsergebnisse führt.

Alle oben genannten Beispiele zeigen den gleichen Trend: Eine Verschiebung des Fettsäurespektrums zu mehr DHA und weniger PA, sowie eine erhöhte Ausbeute an DHA pro Biomasse und Fermentationsvolumen. Die Verschiebung des Fettsäurespektrums macht das gewonnene Öl gesundheitlich wertvoller, da bei gleicher Ölmenge mehr gesunde DHA und weniger ungesunde Palmitinsäure zur Verfügung gestellt wird. Auch für die technische Anwendung z.B. in funktionellen Lebensmitteln ist ein erhöhter DHA-Gehalt von Vorteil, da für den gleichen Effekt weniger Gesamtöl zugegeben werden muss. Die verbesserte Gesamtausbeute (pro Fermentationsvolumen) sowie die erhöhte Konzentration an DHA pro Biomasse liefern wirtschaftliche Vorteile in der Produktion.

Die Verschiebung des Fettsäurespektrums ermöglicht aber auch die Herstellung eines klaren Öls (ohne Eintrübungen durch zuviel Palmitin) ohne eine weitere Abtrennung von Palmitin-Anteilen z.B. durch Winterisation.

## Patentansprüche

1. Verfahren zur fermentativen Gewinnung polyunsaturated fatty acids (PUFAs) enthaltender Öle mit verändertem Fettsäureprofil aus Zellen von Mikroorganismen des Phylums der *Labyrinthulomycota,*
**dadurch gekennzeichnet, dass** dem Fermentationsmedium ein Komplexbildner und/oder ein Flockungsmittel zugegeben wird.

2. Verfahren gemäss Anspruch 1, wobei es sich bei dem Komplexbildner um eine EDTA-Verbindung mit einem oder mehreren anorganischen Gegenionen handelt.

3. Verfahren gemäss Anspruch 1 und/oder 2, wobei es sich bei dem Flockungsmittel um eine Fe(III)-Verbindung handelt.

4. Verfahren gemäss Anspruch 2, wobei die EDTA-Verbindung ausgewählt ist aus der Gruppe enthaltend Na-EDTA, Fe-Na-EDTA und K-EDTA.

5. Verfahren gemäss Anspruch 3, wobei es sich bei der Fe(III)-Verbindung um Fe(III)SO₄ handelt.

6. Verfahren gemäss mindesten einem der Ansprüche 1-5, wobei Na-EDTA, Fe-Na-EDTA, K-EDTA und/oder Fe(III)SO₄ in einer Konzentration von maximal 0.5 mM zugegeben wird.

7. Verfahren gemäss mindestens einem der Ansprüche 1 bis 6, wobei Na-EDTA, Fe-Na-EDTA, K-EDTA und/oder Fe(III)SO₄ in einer Konzentration von 0.1 - 1mM, bevorzugt von 0.1 - 0.4 mM und besonders bevorzugt von 0.2 ± 0.1 mM zugegeben wird.

8. Verfahren gemäss mindestens einem der Ansprüche 1-7, wobei das Verfahren die folgenden Schritte umfasst:
a) Kultivierung der Zellen der Mikroorganismen des Phylums der *Labyrinthulomycota* in einem wässrigen Fermentationsmedium;
b) Optional die Abtrennung der Zellen aus Schritt a) von dem wässrigen Fermentationsmedium zum Erhalt einer Biomasse; und
c) Aufschluss der Zellen aus Schritt b) oder optional direkt aus Schritt a)
d) und Isolation der die PUFAs enthaltenden Öle.

9. Verfahren gemäss mindestens einem der Ansprüche 1-8, wobei die Fermentation bei einer Temperatur von 10°C - 40°C durchgeführt wird.

10. Verfahren gemäss mindestens einem der Ansprüche 1-9, wobei es sich bei den PUFAs um Docosahexaensäure (DHA), Docosapentaensäure (DPA) und/oder Eicosapentaensäure (EPA) handelt.

11. Verfahren gemäss mindestens einem der Ansprüche 1-10, wobei die Mikroorganismen zu der Ordnung der *Thraustochytriales* gehören.

12. Verfahren gemäss Anspruch 11, wobei die Mikroorganismen der Gattung *Japonochytrium, Schizochytrium, Thraustochytrium Althornia, Labyrinthuloides, Aplanochytrium* und/oder *Ulkenia* angehören.

13. Verfahren gemäss Anspruch 12, wobei die es sich bei den Mikroorganismen um Ulkenia SAM 2180 und/oder Schizochytrium SR21 handelt.

14. Verfahren gemäss mindestens einem der Ansprüche 1-13, wobei das wässrige Fermentationsmedium eine oder mehrere Kohlenstoff- sowie eine oder mehrere Stickstoffquellen umfasst.

15. Verfahren gemäss Anspruch 14, wobei es sich bei der Kohlenstoffquelle um Kohlenhydrate wie Glukose, Fruktose Xylose, Saccharose, Maltose, lösliche Stärke, Fucose, Glucosamin, Dextran, Glutaminsäure, Melasse, Glyzerin oder Mannitol, Fette, Öle, Maisquellwasser, Malzextrakt und/oder pflanzliche Hydrolysate handelt.

16. Verfahren gemäss Anspruch 14, wobei es sich bei der Stickstoffquelle um eine natürliche Stickstoffquelle wie Pepton, Hefeextrakt, Fleischextrakt, Proteinhydrolysate, Sojabohnen, eine organische Stickstoffquelle wie Glutamat und Harnstoff und/oder eine anorganische Stickstoffquelle wie Ammoniumacetat, Ammoniumhydrogencarbonat, Ammoniumsulfat oder Ammoniumnitrat handelt.

17. Verfahren gemäss mindestens einem der Ansprüche 1-16, wobei die Fermentation bei einem pH-Wert von 3-8 durchgeführt wird.

18. PUFAs enthaltende Öle, erhältlich mittels eines Verfahrens gemäss mindestens einem der Ansprüche 1-17.

19. Öle gemäss Anspruch 18, wobei die DHA-Konzentration um mehr als 2%, bevorzugt mehr als 3% und besonders bevorzugt mehr als 4% erhöht ist.

20. Öle gemäss Anspruch 18 und/oder 19, wobei die Konzentration von Palmitinsäure (PA) um mehr als 2%, bevorzugt um mehr als 3% und besonders bevorzugt um mehr als 4% reduziert ist.

21. Öle gemäss mindestens einem der Ansprüche 18-20, wobei das Verhältnis von DHA zu PA um mehr als 4%, bevorzugt um mehr als 6% und besonders bevorzugt um mehr als 8% erhöht ist.

22. Öle gemäss mindestens einem der Ansprüche 18-21, wobei das relative Verhältnis von DHA zu Palmitinsäure mindestens 1.3 zu 1 beträgt.

23. Biomasse, erhältlich gemäss einem Verfahren gemäss mindestens einem der Ansprüche 1-17.

24. Biomasse gemäss Anspruch 23, wobei es sich um eine Biotrockenmasse handelt.

25. Verwendung der Öle gemäss mindestens einem der Ansprüche 18-22 als Ergänzungsmittel für die menschliche Ernährung, als Zusatzstoff für Lebensmittel, als Tierfutter und/oder als Medikament.
